(19) Europäisches Patentamt
European Patent Office
Office européen des brevets

(11) EP 1 085 864 B1

(12) **EUROPEAN PATENT SPECIFICATION**

(45) Date of publication and mention of the grant of the patent:
**31.08.2005 Bulletin 2005/35**

(21) Application number: **99928703.0**

(22) Date of filing: **11.06.1999**

(51) Int Cl.[7]: **A61K 31/555**, A61P 31/18

(86) International application number:
**PCT/US1999/013562**

(87) International publication number:
**WO 1999/063947 (16.12.1999 Gazette 1999/50)**

(54) **METHOD OF HIV AND HPV PROPHYLAXIS**

VERFAHREN ZUR PROPHYLAXE VON HIV UND HPV

PROCEDE DE PROPHYLAXIE DU VIH ET DU VPH

(84) Designated Contracting States:
**AT BE CH CY DE DK ES FI FR GB GR IE IT LI LU MC NL PT SE**

(30) Priority: **11.06.1998 US 89250 P**

(43) Date of publication of application:
**28.03.2001 Bulletin 2001/13**

(73) Proprietor: **REDOX PHARMACEUTICAL CORPORATION**
**Greenvale, NY 11548 (US)**

(72) Inventor: **STEWART, Claudia, Cherney**
**Southampton, NY 11968 (US)**

(74) Representative: **Wright, Robert Gordon McRae**
**Elkington and Fife LLP,**
**Prospect House,**
**8 Pembroke Road**
**Sevenoaks,**
**Kent TN13 1XR (GB)**

(56) References cited:
**WO-A-99/56552** **US-A- 5 049 557**
**US-A- 5 106 841** **US-A- 5 756 491**

- **GALASSO, G.J., WHITLEY, R.J., MERIGAN, T.C., EDITORS: "Antiviral Agents and Viral Diseases of Man" 1990 , RAVEN PRESS , NY, USA XP002186694 * page 581 - page 599 ***
- **FIELDS, B.N., EDITOR: "Virology" 1985 , RAVEN PRESS , NY, USA XP002186695 * page 371 - page 382 ***

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 085 864 B1

## Description

### BACKGROUND OF THE INVENTION

The present invention relates to metallo-organic cobalt compounds and their use in the manufacture of a medicament for the prevention of human immunodeficiency virus (HIV) and/or human papillomavirus (HPV) infections.

It has been discovered that certain conditions and diseases, e.g., inflammation, burns, wounds, and diseases caused by bacteria and fungi in mammalian species can be prophylactically treated with certain complexes of cobalt having the structure:

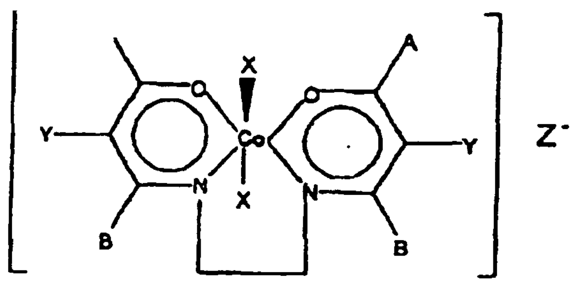

I.

wherein each A may be the same or different and is an alkyl group, a phenyl group or a substituted derivative of a phenyl group;

wherein each Y may be the same or different and is hydrogen, an unbranched alkyl group, a halide or a group having the structure

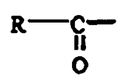

wherein R is hydrogen, an alkoxide group, and alkyl group, or OH;

wherein each B may be the same or different and each is hydrogen or an alkyl group;

wherein each X may be the same or different and each is a water soluble group having weak to intermediate ligand filed strength; and

$Z^-$ is a soluble, pharmaceutically acceptable negative ion.

U. S. Patent 5,142,076, discloses the use of the foregoing described compounds as treatment for viral diseases.

US Patent 5,049,557 discloses the use of compounds similar to those of the invention as antibacterial agents for the prevention of the colonisation of a wound by pathological agents.

US Patents 5,106,841 and 5,756,491 disclose compositions, containing compounds of the invention, having significant activity as antiviral agents.

Virology, B.N. Fields, D.N. Knipe, R.N. Chanock, J.L. Melnick, and R.E. Shope, Raven Press, NY (1985) and Antiviral Agents and Viral Diseases of Man. George J. Galasso. Richard J. Whitley. And Thomas C. Merigan, Ed., 3rd Edition, 1990, Raven Press, NY disclose known viruses of clinical significance to the present application.

Today, virus infections are known to be significant causes of morbidity and mortality in human and veterinary medicine. Many of these diseases are untreatable or the available therapies are not entirely satisfactory and only provide minimal clinical response. New prophylactic treatments would decrease the incidence of these diseases and improve overall health.

### SUMMARY OF THE INVENTION

I have discovered a prophylactic use for the series of compounds having the structure:

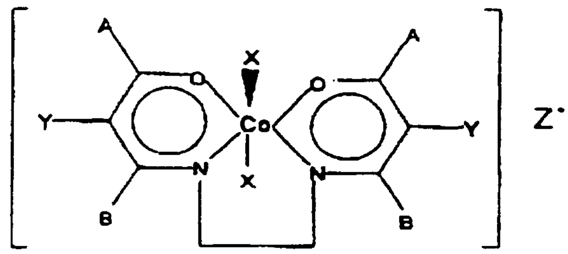

**II.**

wherein

each A may be the same or different and is an alkyl group, a phenyl group or a substituted derivative of a phenyl group;

each Y may be the same or different and is hydrogen, an unbranched alkyl group, a halide or a group having the structure

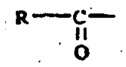

wherein R is hydrogen, an alkoxide group, an alkyl group, or OH;

each B may be the same or different and each is hydrogen or an alkyl group;

Z is a soluble, pharmaceutically acceptable negative ion; and

each X may be the same or different and is an axial ligand selected from the group consisting of moieties having the formula:

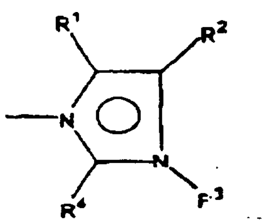

**IIa**

wherein $R^1$, $R^2$, $R^3$, and $R^4$ may be the same or different and maybe hydrogen or lower alkyl having from 1 to 4 carbon atoms; and

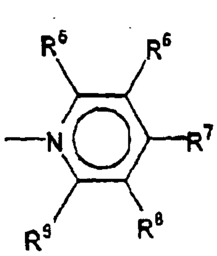

**IIb**

wherein $R^5$, $R^6$, $R^7$, $R^8$ and $R^9$ may be the same or different and may be selected from the group consisting of electron donating groups and electron withdrawing groups,

with the proviso that $R^1$, $R^2$, $R^3$, $R^4$, $R^5$, $R^6$, $R^7$, $R^8$, and $R^9$, are of a sufficiently small size so as not to prohibit the attachment of the axial ligand to the Co atom due to stearic hindrance.

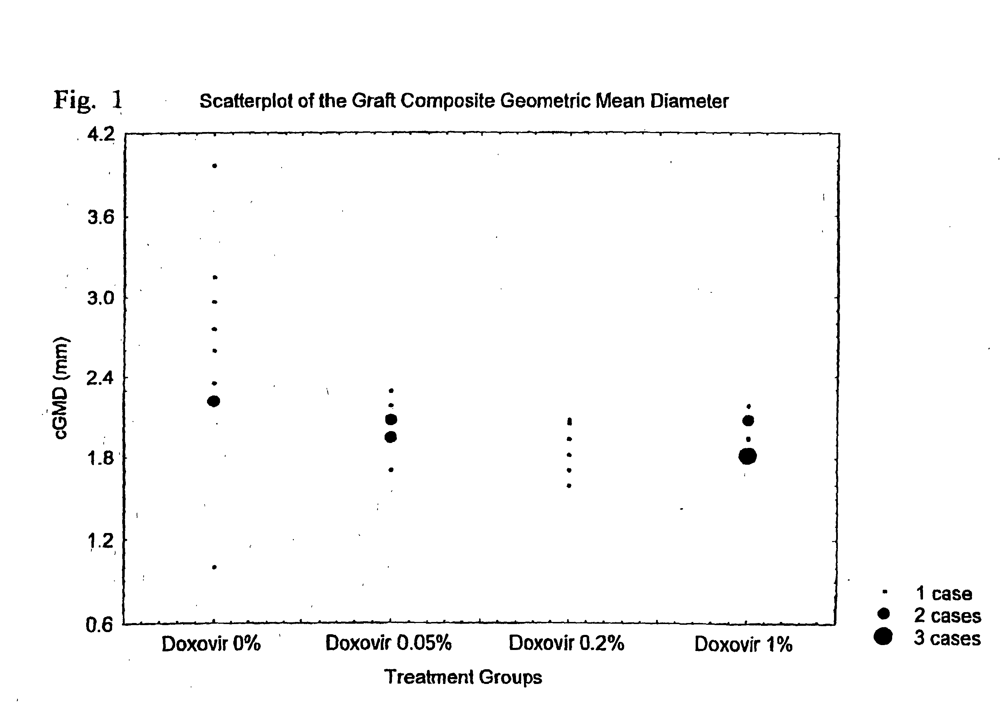

As used herein, the term "axial" when used in conjunction with the term "ligand" refers to the fact that the

ligand is oriented outside the plane of the molecule and has the same meaning as described in connection with Figure 1 of U.S. Patent No. 5,049,557. As used herein, and unless otherwise indicated, an alkyl group means a linear, branched or cyclic alkyl group containing from one to six carbon atoms.

The compounds having the structure of Formula II exhibit prophylactic efficacy when applied as a topical composition to the contact site prior to contact with HIV or HPV, and/or by inactivating HIV or HPV exposed to the composition, and/or by preventing expression of HIV or HPV disease. The compositions of the invention may further be used for antisepsis or disinfection of surfaces, such as surgical tools, or preparations, such as media or blood-derived products.

## BRIEF DESCRIPTION OF THE DRAWINGS

Figure 1 is a scatterplot of the effect of prophylactic treatment of Human Papillomavirus on the size of infected skin grafts in an animal model.

## DETAILED DESCRIPTION OF THE INVENTION

The compounds used in the present invention may be crystallized with numerous counter-anions. Counter-anions which are pharmaceutically acceptable and are water soluble, such as, halide ions, $PF_6^-$ and $BF_4^-$, are preferred. The bromide and chloride salts of the present compounds are the most preferred because they are more water soluble than other salts of the compounds.

As discussed above, A may be an alkyl group, a phenyl group or a substituted derivative of a phenyl group. Preferably, the alkyl group is a $C_1$-$C_5$ group with methyl, ethyl, and butyl groups being particularly preferred. Suitable substituted derivatives of the phenyl group are derivatives wherein each substituent is a halide, an alkyl group or a group having the structure

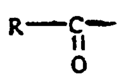

where R is hydrogen, an alkoxide group, an alkyl group or an OH group. To date, the most useful derivatives have proven to be those in which the substiments are halides, or alkyl groups.

Y may be hydrogen, an unbranched alkyl group, a halide or a group having the structure

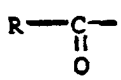

wherein R is hydrogen, an alkoxide group, an alkyl group, or an OH group. In certain embodiments, it is preferred that Y is chlorine, hydrogen atom or a $C_1$-$C_3$ alkyl group. In embodiments where Y has a structure

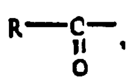,

it is preferred that R is hydrogen a methyl group, or an OH group.

B may be hydrogen or an alkyl group, and preferably is a $C_1$-$C_3$ alkyl group.

X may be imidazole or pyridinyl groups linked to the cobalt atom through a nitrogen of the ring. The imidazole or pyridinyl nuclei may have hydrogen atoms, or electron donating or withdrawing groups substituted thereon.

The electron withdrawing or donating groups which may constitute appendant groups $R^1$, $R^2$, $R^3$, $R^4$, $R^5$, $R^6$, $R^7$ and $R^8$ are those known in the art to exert the specified electron withdrawing or donating effects on aromatic nuclei. Typical of electron donating groups are $NO_2^-$, $Cl^-$, $Br^-$, and the like. The identity of the particular group is not crucial so long as it does not impart properties to the molecule which are detrimental to the desired properties of the compound, e.g., decreased antiviral activity, increased toxicity, and the like. Additionally, the group must not be so large as to prevent the axial ligand to attach to the cobalt atom due to steric effects, e.g., steric hindrance.

Preferably, the groups attached to the imidazole nucleus are alkyl having from one to three carbon atoms. Of

these, methyl and ethyl are most preferred. Preferred are the unsubstituted, 2-methyl, 4-methyl, and 2-ethyl imidazoles and the unsubstituted pyridinyl.

The following Table provides the structures of preferred compounds in accordance with the present invention. Compound 23, disclosed in the U. S. Patent 5,142,076 as exhibiting antiviral activity, is included as a comparison in the examples that follow.

In the following diagram, B is, in each case, methyl, and A, Y, X and $Z^-$ refer to those symbols as used in structure II.

| COMPOUND | Y | X | Z | A |
|---|---|---|---|---|
| 23 | H | $-NH_3$ | Cl | $-CH_3$ |
| 76 | H | 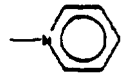 | Br | $-CH_3$ |
| 82 | H | 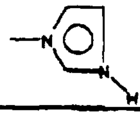 | Cl | $CH_3$ |
| 93 | Cl | 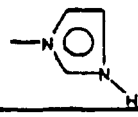 | Br | $-CH_3$ |
| 96 | H | 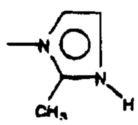 | Br | $-CH_3$ |
| 97 | H | 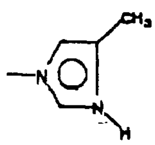 | Br | $-CH_3$ |
| 98 | H | 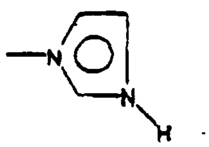 | Br | $C_6H_5$ |
| 100 | Cl | 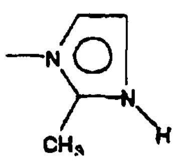 | Br | $-CH_3$ |
| 101 | Cl | 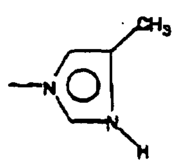 | Br | $-CH_3$ |
| 102 | H | 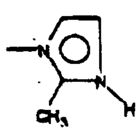 | Cl | $C_6H_5$ |
| 109 | H | 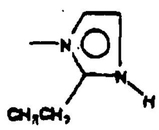 | Cl | $-CH_3$ |

The compositions used in the instant invention comprise a pharmaceutically acceptable carrier and a compound as defined above in an HIV and/or HPV prophylactic effective amount. As used herein, the expressions HIV and/or HPV prophylactic effective amount, dosage or regimen means that amount, dosage or regimen which results in a sufficient concentration of the particular compound at an appropriate site to prevent HIV and/or HPV disease. By appropriate site, it is meant a site which potentially contains HIV and/or HPV or is an area of a subject of potential exposure to HIV and/or HPV disease or is an area of a subject that has been exposed to HIV and/or HPV disease but as a result of such exposure, the subject has not yet acquired HIV or HPV disease. As used herein, the expression acquired HIV or HPV disease means that the subject, in fact, has the disease and can no longer be treated prophylactically to prevent symptoms of the disease and must be treated therapeutically to ameliorate the disease.

The compounds and compositions may be used in preventing infections caused by a variety of HIV or HPV types, such as HIV-1, HIV-2, HPV-1, HPV-2, HPV-3, HPV-4, HPV-6, HPV-7, HPV-10, HPV-11, HPV-16, HPV-18, HPV-31 or HPV-45. Certain compounds within the group may exhibit greater efficacy against specified types as compared with other compounds within the inventive group. Accordingly, the present invention includes the inventive compositions wherein the composition contains a compound as defined hereinabove in a prophylactic amount which is effective against the specific HIV or HPV type.

Known viruses of clinical significance are disclosed in PDR Medical Dictionary, 1st Edition, Williams & Wilkins, pp. 1939-1947, (1995); Virology, B.N. Fields, D.M. Knipe, P.M. Howley, R.M. Chanock, J.L. Melnick, T.P. Monath, B. Roizman and S.E. Straus, Lippincott-Raven Press, N.Y. (1996). See also Antiviral Agents and Viral Diseases of Man, George J. Galasso, Richard J. Whitley, and Thomas C. Merigan, Ed., 4$^{th}$ Edition, Lippincott-Raven Press, N.Y. (1997).

The compounds are particularly effective against, *inter alia*, HIV-1, HIV-2, HPV-1, HPV-2, HPV-3, HPV-4, HPV-6, HPV-7, HPV-10, HPV-11, HPV-16, HPV-18, HPV-31 and HPV-45.

For topical administration, the inventive composition may be placed in a pharmaceutically acceptable saline solution, ointment, salve, cream or the like. The compounds used in the present invention are water soluble, although the degree of solubility may vary from compound to compound, and may be dissolved in a number of conventional pharmaceutically acceptable carriers. Suitable carriers include polar, protic solvents, such as, water, or normal saline. The compounds may also be suspended in a suspension medium that is not miscible with water, for example, petrolatum.

When the compounds of formula II are to be administered by the topical route for prevention of infection, i.e., prophylaxis or disinfection, their concentration in the saline, ointment, salve, creme, or the like can vary from about 0.00005 to about 5% by weight. A preferred concentration range lies between about 0.0005 and about 2% by weight. Typically, the topical composition shows prophylactic effect when applied to the contact site from about 1 hour before contact with the virus to about 6 hours after contact with HIV or HPV. Preferably, the topical composition is applied within five minutes of contact with HIV or HPV. Parti cularly, the inventive compositions can be applied intravaginally for the prevention of sexual transmission of HIV and/or HPV. The topical composition containing the inventive compound could, for example, be coated on a condom or other sexual barrier device.

When the compounds of formula II are to be used for disinfecting liquid preparations, such as, media, blood-derived products or the like, their concentration in the liquid preparations is from about 0.00005 to about 5% by weight. A preferred concentration range lies between about 0.005% and about 2% by weight. A most preferred concentration range lies between about 0.01 % and about 2% by weight.

General methods for the synthesis of the compounds of the present invention are described in U.S. Patent No. 5,049,557, referred to and incorporated by reference hereinabove. As noted therein, the reaction of Co(II) complexes with molar oxygen has been studied extensively (see, R.S. Drago and B. R. Corden, Acc. Chem. Res., 1980, 13, 353 & E. C. Niederhoffer, J. H. Timmons and A.E. Martell, Chem. Rev. 1984, 84, 137). Normally, cobalt (II) forms 2:1 peroxo bridged complexes in aqueous solutions (see E. C. Niederhoffer, J.H. Timmons and A. E. Martell, Chem. Rev. 1984, 84, 137). In recent years, a number of Co(II) complexes have been reported to give 1:1 cobalt-oxygen adducts at room temperature. These complexes usually contain ligands which when bound to Co(II) give rise to a low spin planar geometry. Addition of base and $O_2$ to these complexes leads to the formation of octahedral complexes where the base and the $O_2$ occupy axial positions (see, A. Summerville, R.D. Jones, B.M. Hoffman and F. Basolo, J. Chem. Educ., 1979, 56, 3, 157).

On the basis of measurements utilizing a variety of physical techniques, it is now a well-accepted fact that the most accurate electronic structure description of the Co:$O_2$ moiety is a Co(III) ion bound to $O_2^-$, where the actual amount of Co - $O_2$ electron transfer depends on the nature of the ligand and the donor set (see, A. Summerville, R. D. Jones, B.M. Hoffman and F. Basolo, J. Chem. Educ. 1979, 56, 3 157, & D. Getz, E. Malmud, B. L. Silver and Z. Dori, J. Am Chem. Soc., 1975, 97, 3846). It has been shown that electron transfer increases with increase of the ligand field strength (see, R. S. Drago and B. R. Corden, Acc. Chem. Res., 1980, 13, 353). This can be easily understood from the molecular orbital diagram depicted in Fig. 1 of U. S. Patent 5,049,557 and the description therein.

The following examples illustrate the present invention. The methods used in the examples are described in the following references:

For *in vivo* activity and toxicity of antiviral drugs, see Antiviral Agents and Viral Diseases of Man, *supra*. In

particular, Chapter 3, Preclinical Evaluation of Antiviral Agents; *In vitro* and Animal Model Testing by Dr. Earl R. Kern; and Chapter 6, Major Ocular Viral Infections by Dr. Deborah Paran-Langston.

EXAMPLE 1

Preparation of Prophylactic Compounds

The compounds of the present invention may be prepared by the following general procedure. The cobalt-II complex is prepared by mixing equimolar amounts of the N,N'-bisethylenediimine ligands, e.g., L23 and the like as disclosed in U.S. Patent No. 5,049,557 with cobalt acetate in methanol under nitrogen. About 2.2 equivaleuts of the desired axial ligand is added followed by oxidation. The desired product may then be precipitated by the addition of a saturated aqueous solution of sodium chloride or sodium bromide followed by recrystallization from an ethanol-water solution.

Compound 96 (having bromide as the counterion) was synthesized as follows:

A 3-neck flask equipped with a nitrogen bubbler and a 2 liter dropping funnel was charged with 112 grams (0.5 moles) of the ligand (L23 or N,N'bis-(acerylacetone)ethylene-diimine) in 500 ml of absolute methanol. To the ligand solution is added 125 grams (0.5 moles) of cobalt acetate tetrahydrate dissolved in 1.5 liters of degassed methanol. The reaction mixture is stirred for 2 hours and then refluxed for 15 minutes on a hot water bath. An orange solution results to which 90 grams (1.1 moles) of 2-methyl imidazole dissolved in 100ml of methanol are added. The reaction mixture is exposed to the open air while maintaining vigorous stirring. Ten grams of activated charcoal are added to the stirring mixture and the oxidation is continued overnight.

The mixture is then filtered and 50 grams of sodium bromide dissolved in a minimum amount of water is added to the filtered brown solution. The solution obtained is concentrated and allowed to crystallize. The crude product is recrystallized from hot ethanol-water solution by standing at room temperature or a lower temperature. The purity of the product is checked by elemental analysis, electronic spectra and NMR.

EXAMPLE 2

*In vitro* Assays with HIV

In a study of the prophylactic effects of Compound 96 on Human Immunodeficiency Virus (HIV), virus stock solutions were inactivated by treatment with Compound 96. Stock solutions of viral types NL-HX-ADA having a p24 concentration of 4.1 μg/ml and NL-HX (P122) having a p24 concentration of 3.8 μg/ml were used for the study, along with a stock solution of Compound 96 having a concentration of 20 mg/ml in RPMI media.

Concentrated stock solution of viruses were treated for 1 hr with equal volume of medium containing the drug at the following concentrations;

Table 1.

| Prophylactic Treatments | | |
|---|---|---|
| Initial [Cmpd 96] | | [Cmpd 96] during incubation with virus |
| A. | 20 mg/ml | 10 mg/ml |
| B. | 10 mg/ml | 5 mg/ml |
| C. | 5 mg/ml | 2.5 mg/ml |
| D. | 2.5 mg/ml | 1.25 mg/ml |
| E. | 1.25 mg/ml | 0.6125 mg/ml |
| F. | control - no drug | 0 mg/ml |

After the 1 hr treatment period, the virus-drug solutions were diluted 1:1,000 with complete medium, and then mixed with an equal volume of Peripheral Blood Mononucleocyte (PBMC) cells, for a final dilution of virus and drug of 1:4,000. This resulted in a final virus concentration of 1 ng/ml p24, and the following final drug concentrations:

Table 2.

| Residual concentration of Compound 96 present during incubation with cells | |
|---|---|
| A. | 5 μg/ml |

Table 2. (continued)

| Residual concentration of Compound 96 present during incubation with cells | |
|---|---|
| B. | 2.5 μg/ml |
| C. | 1.25 μg/ml |
| D. | 0.6 μg/ml |
| E. | 0.3 μg/ml |
| F. | 0 |

The infected cultures were then incubated without any washing or other changes in media for 4 days (for the M-tropic NL-HX-ADA type) and 8 days (for the T-tropic NL-HX type). The cultures were then assayed for infection using standard fluorescent focus assays to quantitate infected cells.

Control infections were performed by adding untreated viruses at a p24 concentration of 1 ng/ml into cultures containing the final dilution of the drug, This controlled for any residual effect the diluted drug may have on the health of the PBMC culture and their ability to replicate virus.

For both the T cell-tropic (NL-HX) and macrophage-tropic (NL-HX-ADA) virus, the treated viruses were completely inactivated, even at the lowest concentration of drug tested (0.625 mg/ml). No toxicity or inhibition was seen for the control cultures receiving the diluted drugs, even at the highest concentration tested (5 μg/ml).

Below, the actual numbers of infected cells counted per unit area are reported. No infected cells were detected for any of the drug-treated samples, even when the entire well was scanned, whereas control infection had 800-1200 infected cells per well. This indicates at least a 1,000-fold reduction in infectivity. In fact, this data supports the conclusion that a complete inactivation of virus was achieved.

Table 3.

| Treatment of NL-HX-ADA (macrophage-tropic isolate) Virus | | | | | | |
|---|---|---|---|---|---|---|
| # infected cells/area (-500 cells) | | | | | | |
| Drug conc. (mg/ml): | :no drug | 10 | 5 | 2.5 | 1.25 | 0.625 |
| | 14 | 0 | 0 | 0 | 0 | 0 |
| | 15 | 0 | 0 | 0 | 0 | 0 |
| | 15 | 0 | 0 | 0 | 0 | 0 |
| | 15 | 0 | 0 | 0 | 0 | 0 |
| | 16 | 0 | 0 | 0 | 0 | 0 |
| | 16 | 0 | 0 | 0 | 0 | 0 |
| | 17 | 0 | 0 | 0 | 0 | 0 |
| | 17 | 0 | 0 | 0 | 0 | 0 |
| | 17 | 0 | 0 | 0 | 0 | 0 |
| | 16 | 0 | 0 | 0 | 0 | 0 |
| Average # infected cells/area | 15.8 | 0 | 0 | 0 | 0 | 0 |
| Control culture: | | | | | | |
| | 14 | 15 | 16 | 17 | 16 | 17 |
| | 17 | 16 | 17 | 18 | 15 | 16 |
| | 16 | 17 | 15 | 17 | 16 | 16 |
| | 17 | 16 | 15 | 15 | 17 | 17 |
| | 16 | 15 | 17 | 17 | 17 | 18 |
| | 15 | 17 | 16 | 16 | 16 | 16 |
| | 17 | 16 | 16 | 16 | 16 | 16 |
| | 16 | 16 | 18 | 16 | 18 | 16 |
| | 16 | | | | | |
| | 16 | | | | | |
| Average # infected cells/area | 16.00 | 16.00 | 16.25 | 16.50 | 16.38 | 16.50 |

Table 4.

| Virus: NL-HX (T cell-tropic isolate) Virus | | | | | | |
|---|---|---|---|---|---|---|
| # infected cells/area (~500 cells) | | | | | | |
| Drug cone. (mg/ml): | no drug | 10 | 5 | 2.5 | 1.25 | 0.625 |
| | 11 | 0 | 0 | 0 | 0 | 0 |
| | 10 | 0 | 0 | 0 | 0 | 0 |
| | 10 | 0 | 0 | 0 | 0 | 0 |
| | 9 | 0 | 0 | 0 | 0 | 0 |
| | 11 | 0 | 0 | 0 | 0 | 0 |
| | 10 | 0 | 0 | 0 | 0 | 0 |
| | 10 | 0 | 0 | 0 | 0 | 0 |
| | 11 | 0 | 0 | 0 | 0 | 0 |
| | 11 | 0 | 0 | 0 | 0 | 0 |
| | 11 | 0 | 0 | 0 | 0 | 0 |
| Average # infected cells/area | 10.4 | 0 | 0 | 0 | 0 | 0 |
| Control culture: | | | | | | |
| | 10 | 10 | 10 | 10 | 9 | 11 |
| | 11 | 10 | 10 | 10 | 8 | 10 |
| | 8 | 9 | 8 | 11 | 11 | 10 |
| | 10 | 10 | 8 | 10 | 10 | 10 |
| | 10 | 11 | 9 | | | |
| | 8 | 9 | 8 | | | |
| | 9 | 9 | 8 | | | |
| | 9 | 10 | 9 | | | |
| Average # infected cells/area | 9.4 | 9.8 | 8.8 | 10.3 | 9.5 | 10.3 |

EXAMPLE 3

Titration of Anti-HIV Effect of Ctc 96

General Methodology:

Stock virus: NL-HX-ADA ~5.5 µg/ml p24 (This is a molecularly cloned macrophage-tropic type of HIV-1). Virus was used to infect cells at 1:4,000 final dilution (~1.4 ng/ml p24).

Stock CTC-96: 10 mg/ml prepared directly in RPMI 1640

Method: Concentrated stock solution of virus was treated for 1 hour with equal volume of medium containing the drug at the indicated concentrations (1:2 dilution). The virus/drug mixture was the diluted 1,000-fold (1:2,000 dilution) and then added to an equal volume of PHA-activated humans PBMCs (1:4,000 final dilution) to allow virus replication to occur. The extent of infection was measured after 4 days by a fluorescent focus assay and compared to that of a control culture not containing any drug, No residual toxicity of CTC-96 was detected under these conditions (highest concentration = 2.5 ng/ml).

Experiment 1:

Table 5.

| Extended dose responses for inactivation of HIV-1 by CTC96 | | | | |
|---|---|---|---|---|
| Batch | Initial [CTC96] mg/ml | [CTC96] during incubation with virus mg /ml | [CTC96] after final dilution ng/ml | % Neutralization |
| A | 10 | 5 | 2.5 | 100 |
| B | 5 | 2.5 | 1.25 | 100 |
| C | 2.5 | 1.25 | 0.61 | 100 |
| D | 1.25 | 0.625 | 0.31 | 100 |
| E | 0.6 | 0.312 | 0.156 | 83 |
| F | 0.3 | 0.156 | 0.078 | 63 |
| G | 0.156 | 0.078 | 0.039 | 55 |
| H | 0.078 | 0.039 | 0.019 | 18 |
| I | 0.039 | 0.019 | 0.009 | 1 |
| J | 0.019 | 0.009 | 0.0045 | 2 |
| K | 0.009 | 0.0045 | 0.0023 | 1 |

Experiment 2

Time course of anti-HIV effect of CTC96

Using similar conditions as described above, virus was incubated with 0.625 mg/ml CTC96 for the indicated time period before 1,000-fold dilution and addition to cells.

Virus was incubated four different concentrations of CTC96 for the indicated time periods, and then the extent of inactivation tested as previously described. Numbers in parentheses indicate results obtained in the initial analysis for the lowest dose (0.625 μg/ml) of CTC96.

Table 6.

| Neutralization of HIV by incubation with CTC 96 | | | | |
|---|---|---|---|---|
| Contact time (minutes) | % Neutralization for [CTC96] - | | | |
| | 5 μg/ml | 2.5 μ/ml | 1.25 μg/ml | 0.625 μg/ml |
| 64 | 100 | 100 | 94 | 80 (100) |
| 32 | 98 | 86 | 78 | 65 (76) |
| 16 | 78 | 72 | 61 | 56 (64) |
| 8 | 65 | 47 | 42 | 41 (56) |
| 4 | 52 | 25 | 17 | 25 (43) |
| 2 | 37 | 21 | 4 | 15 (7) |

EXAMPLE 4

Prophylactic Activity of CTC 96 against Human Papillomavirus Type 11

The activity of CTC 96 on human papillomavirus type 11 (HPV-11) prior to infection was tested by using a subcutaneous human xenograft infection model in the severe combined immunodeficiency (SCID) mouse.

**METHODS**

A. Protocol

Three different concentrations, 1%, 0.2% and 0.05%, of CTC 96 and a control (normal saline) were evaluated in HPV-11-infected internal human xenograft in the SCID mouse. The viral suspension was exposed for one hour with one of the four CTC 96 concentrations before being used to infect the human foreskin grafts.

Each experiment was done in quadruplicate. Twelve mice were used in each replicate. For each replicate, the four treatment groups were made of 3 mice (3 animals per cage). Each mouse received under the skin of each flank one HPV-11-infected foreskin fragment. Two foreskin donors were used, one for the fragments implanted under the left flank and a different one for the fragments implanted under the right flank. The HPV-11-infected grafts were left to grow for 12 weeks, while the mice weights were monitored every other week. At the end of the 12 weeks, the animals were sacrificed and the grafts recovered, measured and processed.

B. Experimental Design

Six to 7 week old male *scid*/*scid* mice were used

Table 7 -

| Experimental Design | | | | |
|---|---|---|---|---|
| Treatment | Replicate # | | | |
| | 1 | 2 | 3 | 4 |
| CTC 96 1 % (Group 1) | 3 mice | 3 | 3 | 3 |
| CTC 96 0.2% (Group 2) | 3 | 3 | 3 | 3 |
| CTC 96 0.05% (Group 3) | 3 | 3 | 3 | 3 |
| CTC 96 0% (saline, control) (Group 4) - | 3 | 3 | 3 | 3 |

C. Drug Treatment

CTC 96 was applied to the virus directly, prior to infection. Just prior to exposure of the virus to CTC 96, 750 µl of normal saline was added to a vial containing 15 mg of dry CTC 96. The resulting solution contained 2% of CTC 96. This solution was diluted as indicated in Table 8, so that once mixed with the virus, the final concentration was 0%, 0.05%, 0.2%, or 1%.

CTC 96 was incubated for 1 hour at 37°C with the viral suspension, which was then used to infect the human foreskin fragments.

Table 8.

| Preparation of the viral Suspensions | | | | |
|---|---|---|---|---|
| | Dilution 1 | Dilution 2 | Dilution 3 | Dilution 4 |
| Solute | 15 mg of dry CTC 96 | 150 µl of dilution 1 | 30 µl of dilution 1 | - |
| Normal saline | 750 µl | 600 µl | 570 µl | 700 µl |
| Volume of the aliquot of the corresponding dilution | 500 µl | 500 µl | 500 µl | 500 µl |
| HPV-11 viral suspension (lysate 4/90; 1:19 dilution) | 500 µl | 500 µl | 500 µl | 500 µl |
| Final CTC 96 concentration (w/v) | 1% | 0.2% | 0.05% | 0% |
| Treatment Group | 1 | 2 | 3 | 4 |

D. Grafting

Neonatal foreskins from routine circumcision were collected at the nursery of a local hospital and placed in

transport medium (Minimum Essential medium, perucillin 25,000 U/ml, streptomycin 25 mg/mL). In a Petri dish, the foreskin's occluded side was removed using a scalpel, leaving the exposed skin prepared as a split-thickness graft. The foreskin was punched out using a 3 mm biopsy punch. One fragment was fixed in buffered formalin to serve as control in the histologic and immunocytochemical analyses. 3-4 fragments were snap frozen in liquid nitrogen, to serve as controls in the RT-PCR assay. The other fragments were split equally into four groups. Each group of fragments was dispensed in one of the vials containing 500 µl of pretreated viral suspension.

Two foreskin donors collected within 24 hours of the procedure, were used in each replicate experiment. Each viral suspension received the foreskin fragments of both foreskin donors. To recognize the foreskin donors, in each experiment we used a white baby and a black baby. The fragments were vortexed in the suspension and left to incubate for 60 minutes at 37°C before being grafted.

For the grafting, the mouse was anesthetized in an anesthesia chamber with 5% halothane to 2 liters of oxygen, until no corneal or pedal reflexes could be elicited. The animal was removed from the chamber, and the halothane adjusted to 2 - 2.5% to 1 liter of oxygen for continued administration via nose cone for the duration of the surgical procedure. The animals ears were notched for identification. The back skin was prepped with a povidone iodine swab. A 1 cm vertical incision of the skin was made on the flank with scissors. Through that incision, the subcutis was bluntly dissected with closed scissors caudad to the wound. One foreskin fragment from the white donor was inserted in the caudad pocket, epidermis facing the mouse's skin, mucosal side in contact with the musculo-fascial plane (panniculus camosus) of the mouse. The incision was closed with two metal clips. The other side was grafted in the same manner with one foreskin fragment from the black donor.

E. Monitoring, Euthanasia and Graft Collection

The animals were monitored weekly. They were weighed at the time of grafting, and every other week during the 12 weeks of the experiment.

Animals were to be prematurely euthanitized if during monitoring any of the following criteria were met:

- greater than 10% weight loss;
- bleeding or bruising;
- inability to maintain righting reflex;
- inability to move about;
- inability to eat or drink;
- tumor greater than 30 % of the animal's body weight.

The animals were otherwise sacrificed by cervical dislocation 12 weeks after graft implantation. Length, width, and height of the graft were measured and recorded. The graft were then removed and split in at least two parts. One part was fixed in buffered formalin. The other part was either left as is, or subdivided for subsequent analyses in as many fragments as size permitted. These fragments were placed in a sterile labelled vial and frozen in liquid nitrogen before being transferred to a -80°C freezer.

F. Premature Deaths

Those animals dying unexpectedly before the date of the scheduled euthanasia had their grafts measured and fixed in formalin, if body decomposition was not too advanced.

G. Statistical Methods

1. Data Management

Patient, animal, and experimental data were entered in a database maintained on a personal computer. Data handling and analysis was done primarily with the statistical software STATISTICA/5.1w (Statsoft Inc.), StatXact (Cytel Corp. Cambridge, MA) software was used for the statistical analysis by exact methods. The Minitab software (Minitab Inc.) was used for the randomization. The statistical power calculations were carried out with the software PASS 6.0 for Windows (NCSS, Kaysville, UT).

2. Design and Analysis of the Primary endpoint (Graft Size)

We conducted the Efficacy studies using a factorial design. The two factors were Treatment (fixed factor) and, as a concomitant variable, the Replicate factor to take into account the well-established variability introduced by the

foreskin donor. The Replicate factor was treated as a fixed factor since it is used for adjustment purposes in the specific experiment. Therefore, the analysis was that of a 2-way crossed, between-subjects ANOVA model. There were 3 replications per cell, corresponding to the 3 animals in a cage.

The cages were sequentially numbered upon arrival in the xenograft facility. Treatment was assigned to the cages from a list of random numbers generated for each replicate and matched to the case sequence number.

For each mouse, at the end of the experiment, we calculated a composite geometric mean diameter (cGMD) of the grafts, which was chosen before the experiment as our primary endpoint:

$$cGMD = (\sqrt[3]{(\text{length x weidth x height})_{\text{left side}}} + \sqrt[3]{(\text{length x width x height})_{\text{right side}}})/2$$

In case one of the two grafts was missing, the denominator was one and if both grafts were missing, then a cCMD was not calculated for that particular animal.

Pairwise comparisons between the CTC 96-treated groups and the vehicle-treated group was done using the Tukey Honest Significant Difference test, modified by Spjotvoll & Stoline for unequal n's. We also conducted an analysis for linear or quadratic trend across the different concentrations of CTC 96.

For $\alpha = 0.05$ and we had calculated a statistical power of 0.80 to detect an effect size of 0.59. The effect size being defined as the difference between the largest and smallest mean cGMDs divided by the within-cell standard deviation.

In all statistical analyses two-sided p values equal or less than 0.05 were considered significant.

RESULTS

A. Animals

Forty-eight animals were grafted according to protocol. Seven animals died or had to be sacrificed before the end of the experiment. The monitored the mortality of the animals in the different groups was monitored and did not detect a significant imbalance that would have led to the premature euthanasia of the animals. This mouse mortality rate is not excessive. SCID mice are fragile, and a major cause of mortality is the spontaneous development in 15% of animals aged 4 to 17 months of thymic lymphomas that readily metastasize.

B. Grafts

82 grafts were implanted in a the 41 mice that survived until the end of the experiment and 77 (94 %) were present at euthanasia. Table 9 summarizes the distribution of the grafts present at euthanasia according to treatment groups. Three mice had only one surviving graft. Unfortunately, the only mouse with no surviving grafts belonged to the CTC 96 0% group of the second replicate, a group that had already lost two animals from premature deaths. Consequently, there was no control group for the second experiment. Therefore the efficacy analysis of CTC 96 that is presented is based on replicates 1, 3 and 4. All animals (4 replicates) are included in the Toxicity Analysis.

Table 9 -

| Grafts present at Euthanasia | | | | | | |
|---|---|---|---|---|---|---|
| Replicate | Treatment Group | | Number of surviving mice with the following number of grafts | | | Row Total |
| | | | 0 | 1 | 2 | |
| 1 | CTC 96 | 0% | 0 | 0 | 3 | 3 |
| | CTC 96 | 0.05% | 0 | 0 | 3 | 3 |
| | CTC 96 | 0.2% | 0 | 0 | 2 | 2 |
| | CTC 96 | 1% | 0 | 0 | 3 | 2 |
| | | **Total** | **0** | **0** | **11** | **11** |

Table 9 - (continued)

| Grafts present at Euthanasia | | | | | | |
|---|---|---|---|---|---|---|
| Replicate | Treatment Group | | Number of surviving mice with the following number of grafts | | | Row Total |
| | | | 0 | 1 | 2 | |
| 2 | CTC 96 | 0% | 1 | 0 0 | | 1 |
| | CTC 96 | 0.05% | 0 | 1 | 2 | 3 |
| | CTC 96 | 0.2% | 0 | 0 | 2 | 2 |
| | CTC 96 | 1% | 0 | 0 | 3 | 3 |
| | | **Total** | **1** | **1** | **7** | **9** |
| 3 | CTC 96 | 0% | 0 | 1 | 2 | 3 |
| | CTC 96 | 0.05% | 0 | 0 | 3 | 3 |
| | CTC 96 | 0.2% | 0 | 0 | 2 | 2 |
| | CTC 96 | 1% | 0 | 0 | 3 | 3 |
| | | **Total** | **0** | **1** | **10** | **11** |
| 4 | CTC 96 | 0% | 0 | 0 | 3 | 3 |
| | CTC 96 | 0.05% | 0 | 0 | 2 | 2 |
| | CTC 96 | 0.2% | 0 | 0 | 2 | 2 |
| | CTC 96 | 1% | 0 | 1 | 2 | 3 |
| | | **Total** | **0** | **1** | **9** | **10** |
| Column Total | | | **1** | **3** | **37** | **41** |

C. Effect of CTC 96 on Graft Size

The composite Geometric Mean Diameter of the grafts was our primary endpoint in this evaluation of CTC 96. Table 10 provides the summary statistics of this measurement. The highest mean (or median) cGMD was in the group where the infecting virus was treated with saline only (CTC 96 0% group). Figure 1 displays the cGMDs of the four treatment groups.

Table 10 -

| Summary Statistics on the Composite Geometric Mean Diameters (cGMD) of the Grafts (mm) | | | | | | |
|---|---|---|---|---|---|---|
| Treatment Groups | Means | N | Standard Deviations | Lower Quartile | Median | Upper Quartile |
| CTC 96 0% | 2.58 | 9 | .808 | 2.22 | 2.89 | 2.96 |
| CTC 96 0.05% | 2.02 | 8 | .179 | 1.94 | 2.01 | 2.13 |
| CTC 96 0.2% | 1.86 | 6 | .196 | 1.70 | 1.88 | 2.05 |
| CTC 96 1% | 1.95 | 9 | .139 | 1.82 | 1.94 | 2.08 |
| All | 2.13 | 32 | .52 | 1.83 | 2.07 | 2.22 |

Table 11 shows the statistical analysis by ANOVA of these results. The graft size varied significantly according to the treatment applied to the viral inoculum ($p = 0.004$). Furthermore, there was a dose-response effect ($p = 0.002$). A pairwise comparison of the CGMDs in the treatment groups (Table 12) establishes that regardless of the CTC 96 concentration there was a significant effect on the infectivity of HPV-11 when compared to the control (no CTC 96).

Table 11 -

| ANOVA of the Effects on Graft Composite Geometric Diameter (cGMD) | | | | | | |
|---|---|---|---|---|---|---|
| Effect | df Effect | MS Effect | df Error | MS Error | Fn | p level |
| Treatment* | 3 | 0.8690 | 20 | 0.1438 | 6.0467 | 0.0043 |
| Replicate | 2 | 0.4941 | 20 | 0.1438 | 3.437 | 0.052 |
| Treatment x Replicate Interaction | 6 | 0.2888 | 20 | 0.1438 | 2.009 | 0.11 |

* There was a dose-response effect (p = 0,0023; by test for linear trend)

Table 12 -

| p-values of the Pairwise Comparisons of the cGMDs between Treatment Groups (Spjotvoll & Stoline test) | | | | | |
|---|---|---|---|---|---|
| Treatment Groups | | CTC 96 0% | CTC 96 0.05% | CTC 96 0.2% | CTC 96 1 % |
| CTC 96 | 0% | - | **0.046** | **0.019** | **0.010** |
| CTC 96 | 0.05% | **0.046** | - | 0.85 | 0.96 |
| CTC 96 | 0.2% | **0.019** | 0.85 | - | 0.98 |
| CTC 96 | 1% | **0.010** | 0.96 | 0.98 | - |

D. Effect of CTC 96 HPV-11 Treatment on Mouse Mortality

Regardless of the endpoint used, number of deaths (p = 0.17: Table 12) or length of survival (p = 0.11; Table 13), there were no differences among the treatment groups.

Table 13 -

| Mouse Mortality during the Experiment | | | | |
|---|---|---|---|---|
| Mouse Status at the end of the Experiment | CTC 96 0% | CTC 96 0.05% | CTC 96 0.2 % | CTC 96 1 % |
| Alive | 10 | 11 | 8 | 12 |
| Dead | 2 | 1 | 4 | 0 |
| **Total** | **12** | **12** | **12** | **12** |
| p = 0.1745: by Fisher-Freeman-Halton exact test | | | | |

Table 14 -

| Mouse Survival (days) Summary Statistics | | | | | | |
|---|---|---|---|---|---|---|
| Treatment Groups | Means | N | Standard Deviations | Lower Quartile | Median* | Upper Quartile |
| CTC 96 0% | 78.67 | 12 | 14.63 | 84.00 | 84.00 | 84.00 |
| CTC 96 0.05% | 83.167 | 12 | 2.89 | 84.00 | 84.00 | 84.00 |
| CTC 96 0.2% | 69.83 | 12 | 24.52 | 57.50 | 84.00 | 84.00 |
| CTC 96 1% | 84.00 | 12 | 0.00 | 84.00 | 84.00 | 84.00 |
| **All Groups** | **78.92** | **48** | **15.00** | **84.00** | **84.00** | **84.00** |

*p = 0.11: by Kruskal-Wallis test

E. Effect of CTC 96 HPV-11 Treatment on Mouse Weight Changes

There was no effect of HPV-11 treatment by CTC 96 on the weight grains of the mice during the experiment (p = 0.23).

Table 15 -

| Mouse Weight Changes (%) during the Experiment - Summary Statistics | | | | | | |
|---|---|---|---|---|---|---|
| Treatment Groups | Means | N | Standard Deviations | Lower Quartile | Median* | Upper Quartile |
| CTC 96 0% | 6.19 | 9 | 9.57 | 3.33 | 8.28 | 12.76 |
| CTC 96 0.05% | 13.12 | 8 | 3.89 | 12.32 | 14.16 | 15.62 |
| CTC 96 0.2% | 15.77 | 6 | 7.71 | 11.48 | 11.77 | 24.80 |
| CTC 96 1% | 9.96 | 9 | 5.08 | 5.926 | 9.00 | 12.40 |
| **All Groups** | **10.78** | **32** | **7.47** | **6.56** | **11.51** | **14.79** |

*p = 0.23: by Kruskal-Wallis test

The effect of CTC 96 on the infectivity of HPV-11 was evaluated in the human xenograft SCID mouse model. The results were analyzed for an effect of CTC 96 on graft size. Although the analysis of the experiment was compromised by the inability to use the results of one of the four replicate experiments, the results were clear: CTC 96 regardless of the concentration used, limited the HPV-11-inducted growth of the grafts. Even the 0.05 % concentration was effective. It is impossible to determine at this stage of the analysis the concentration below which CTC 96 loses its effect on graft size. This question might be answered using other endpoints, histology, capsid protein expression (immunocytochemistry), and viral transcription (RT-PER). This analysis is in progress.

CTC 96 has no effect on the animal's mortality or weight gain. This was expected since the drug was not directly administered to the animals. It was used only to inactivate HPV-11 *in vitro.*

## Claims

**1.** Use of a compound of formula II for are manufacture of a composition for the prevention of Human Immunodeficiency Virus infection:

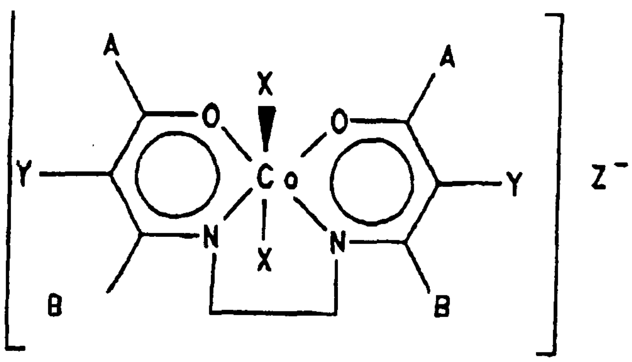

II

wherein each

A may be the same or different and is an alkyl group, a phenyl group or a substituted derivative of a phenyl group;

Y may be the same or different and is hydrogen, an unbranched alkyl group, a halide or a group having the structure

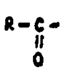

wherein R is hydrogen, an alkoxide group, an alkyl group, or OH;

B may be the same or different and each is hydrogen or an alkyl group;

$Z^-$ is a soluble, pharmaceutically acceptable negative ion, and

X may be the same or different and is an axial ligand selected from the group consisting of moieties having the formula:

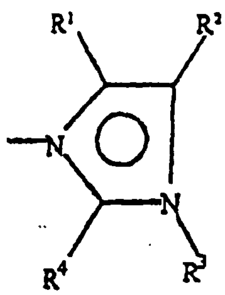

II a

wherein $R^1$, $R^2$, $R^3$, and $R^4$ may be the same or different and may be hydrogen or lower alkyl having from 1 to 4 carbon atoms;

with the proviso that $R^1$, $R^2$, $R^3$, and $R^4$ are of a sufficiently small size so as not to prolubit the attachment of the axial ligand to the Co atom due to steric hindrance.

**2.** Use according to claim 1 wherein the compound is from about 0.00005 to about 5% by weight of the composition.

**3.** Use according to claim 1 wherein the compound is from about 0.005 to about 5% by weight of the composition.

**4.** Use according to claim 1 wherein the compound is from about 0.005 to about 2% by weight of the composition.

**5.** Use according to claim 1 wherein the compound is from about 0.01 to about 2% by weight of the composition.

**6.** Use according to claim 1 wherein the composition is in the form of a pharmaceutically acceptable saline solution, ointment, salve, creme, or the like.

**7.** Use according to claim 1 wherein the composition is applied to that site which is exposed to the Human Immunodeficiency Virus.

**8.** Use according to claim 7 wherein the composition is applied from about 1 hour before to about 6 hours after exposure to the Human Immunodeficiency Virus.

**9.** Use according to claim 7 wherein the composition is applied from about 5 minutes before to about 5 minutes after exposure to the Human Immunodeficiency Virus.

**10.** Use according to claim 1 wherein the Human Immunodeficiency Virus is STRAINS???.

**11.** Use according to claim 1 wherein the compound is Compound 96.

**12.** Use according to claim 1 wherein the composition is applied to an applicator

**13.** Use according to claim 12 wherein the applicator is a condom.

**14.** A method for disinfecting a liquid containing a Human Immunodeficiency Virus comprising adding to the liquid a composition comprising a Human Immunodeficiency Virus prophylactic effective amount of a compound having

the structure

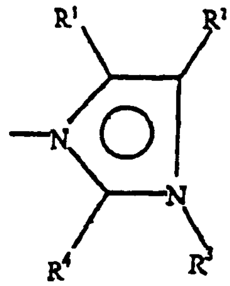

IIa

wherein $R^1$, $R^2$, $R^3$, and $R^4$ may be the same or different and may be hydrogen or lower alkyl having from 1 to 4 carbon atoms;
with the proviso that $R^1$, $R^2$, $R^3$, and $R^4$ are of a sufficiently small size so as not to prohibit the attachment of the axial ligand to the Co atom due to steric hindrance.

**15.** The method of claim 14 wherein the compound is added in an amount of about 0.00005 to about 5% by weight of the liquid.

**16.** The method of claim 14 wherein the compound is added in an amount of about 0.005 to about 5% by weight of the liquid.

**17.** The method of claim 14 wherein the compound is added in an amount of about 0.005 to about 2% by weight of the liquid.

**18.** The method of claim 14 wherein the compound is added in an amount of about 0.01 to about 2% by weight of the liquid.

**19.** The method of claim 14 wherein the liquid is a growth media or a blood-derived product.

**20.** Use of a compound of formula II for are manufacture of a composition for are prevention of Human Papillomavirus infection

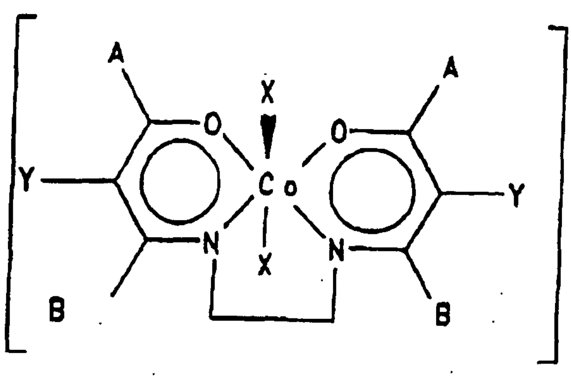

II

wherein each

A may be the same or different and is an alkyl group, a phenyl group or a substituted derivative of a phenyl group;
Y may be the same or different and is hydrogen, an unbranched alkyl group, a halide or a group having the structure

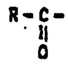

wherein R is hydrogen, an alkoxide group, an alkyl group, or OH;

B may be the same or different and each is hydrogen or an alkyl group;

$Z^-$ is a soluble, pharmaceutically acceptable negative ion, and

X may be the same or different and is an axial ligand selected from the group consisting of moieties having the formula:

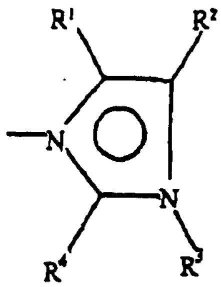

IIa

wherein $R^1$, $R^2$, $R^3$, and $R^4$ may be the same or different and may be hydrogen or lower alkyl having from 1 to 4 carbon atoms;

with the proviso that $R^1$, $R^2$, $R^3$, and $R^4$ are of a sufficiently small size so as not to prohibit the attachment of the axial ligand to the Co atom due to steric hindrance.

**21.** Use according to claim 20 21 wherein the compound is from about 0.00005 to about 5% by weight of the composition.

**22.** Use according to claim 20 wherein the compound is from about 0.005 to about 5% by weight of the composition.

**23.** Use according to claim 20 wherein the compound is from about 0.005 to about 2% by weight of the composition.

**24.** Use according to claim 20 wherein the compound is from about 0.01 to about 2% by weight of the composition.

**25.** Use according to claim 20 wherein the composition is in the form of a pharmaceutically acceptable saline solution, ointment, salve, creme, or the like.

**26.** Use according to claim 20 wherein the composition is applied to that site which is exposed to the Human Papillomavirus.

**27.** Use according to claim 16 wherein the composition is applied from about 1 hour before to about 6 hours after exposure to the Human Papillomavirus.

**28.** Use according to claim 26 wherein the composition is applied from about 5 minutes before to about 5 minutes after exposure to the Human Papillomavirus.

**29.** Use according to claim 20 wherein the Human Papillomavirus is selected from the group cvonsisting of HPV-1, HPV-2, HPV-3, HPV-4, HPV-6, HPV-7, HPV-10, HPV-11, HPV-16, HPV-18, HPV-31 or HPV-45.

**30.** Use according to claim 20 wherein the compound is CTC 96.

**31.** Use according to claim 20 wherein the composition is applied to an application coated with the composition.

**32.** Use according to claim 31 wherein the applicator is a condom.

**33.** A method for disinfecting a liquid containing a Human Papillomavirus comprising adding to the liquid a composition

comprising a Human Papillomavirus prophylactic effective amount of a compound having the structure.

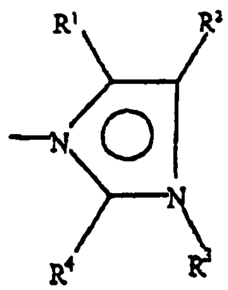

I I a

wherein $R^1$, $R^2$, $R^3$, and $R^4$ may be the same or different and may be hydrogen or lower alkyl having from 1 to 4 carbon atoms;
with the proviso that $R^1$, $R^2$, $R^3$, and $R^4$ are of a sufficiently small size so as not to prohibit the attachment of the axial ligand to the Co atom due to stearic hindrance.

**34.** The method of claim 33 wherein the compound is added in an amount of about 0.00005 to about 5% by weight of the liquid.

**35.** The method of claim 33 wherein the compound is added in an amount of about 0.005 to about 5 % by weight of the liquid.

**36.** The method of claim 33 wherein the compound is added in an amount of about 0.005 to about 2% by weight of the liquid.

**37.** The method of claim 33 wherein the compound is added in an amount of about 0.01 to about 2% by weight of the liquid.

**38.** The method of claim 33 wherein the liquid is a growth media or a blood-derived product.

**Patentansprüche**

**1.** Verwendung einer Verbindung der Formel II zur Herstellung einer Zusammensetzung zur Prävention einer Infektion mit HIV (humanem Immunschwächevirus):

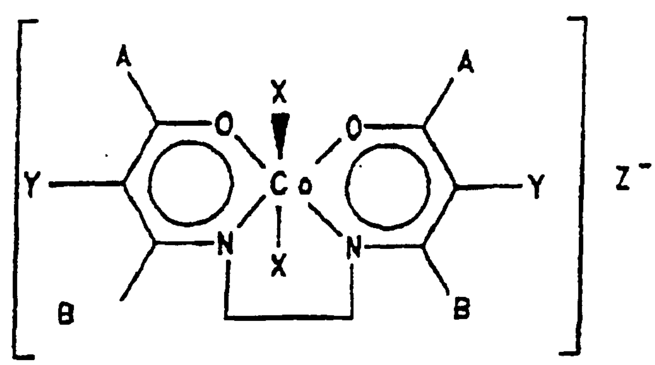

II

wobei jeweils

A xgleich oder verschieden sein können und eine Alkylgruppe, eine Phenylgruppe oder ein substituiertes Derivat einer Phenylgruppe bedeuten;

Y gleich oder verschieden sein können und Wasserstoff, eine unverzweigte Alkylgruppe, ein Halogenid oder eine Gruppe mit der Struktur

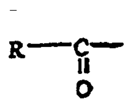

bedeuten, wobei R Wasserstoff, eine Alkoxidgruppe, eine Alkylgruppe oder OH ist;

B gleich oder verschieden sein können und jeweils Wasserstoff oder eine Alkylgruppe sind;

$Z^-$ ein lösliches, pharmazeutisch annehmbares negatives Ion ist; und

X gleich oder verschieden sein können und einen axialen Liganden bedeuten, der aus der Gruppe ausgewählt ist, die aus Struktureinheiten mit der Formel

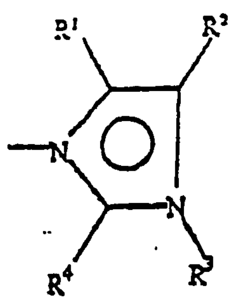

IIa

besteht, wobei $R^1$, $R^2$, $R^3$ und $R^4$ gleich oder verschieden sein können und Wasserstoff oder Niederalkyl mit 1 bis 4 Kohlenstoffatomen sein können;
mit der Maßgabe, dass $R^1$, $R^2$, $R^3$ und $R^4$ ausreichend klein sind, so dass sie die Bindung des axialen Liganden an das Co-Atom nicht aufgrund von sterischer Hinderung verhindern.

**2.** Verwendung gemäß Anspruch 1, wobei die Verbindung etwa 0,00005 bis etwa 5 Gew.-% der Zusammensetzung ausmacht.

**3.** Verwendung gemäß Anspruch 1, wobei die Verbindung etwa 0,005 bis etwa 5 Gew.-% der Zusammensetzung ausmacht.

**4.** Verwendung gemäß Anspruch 1, wobei die Verbindung etwa 0,005 bis etwa 2 Gew.-% der Zusammensetzung ausmacht.

**5.** Verwendung gemäß Anspruch 1, wobei die Verbindung etwa 0,01 bis etwa 2 Gew.-% der Zusammensetzung ausmacht.

**6.** Verwendung gemäß Anspruch 1, wobei die Zusammensetzung in Form einer pharmazeutisch annehmbaren Kochsalzlösung, einer Salbe, eines Balsams, einer Creme oder dergleichen vorliegt.

**7.** Verwendung gemäß Anspruch 1, wobei die Zusammensetzung auf diejenige Stelle aufgetragen wird, die dem HIV ausgesetzt ist.

**8.** Verwendung gemäß Anspruch 7, wobei die Zusammensetzung etwa 1 Stunde vor bis etwa 6 Stunden nach dem Kontakt mit dem HIV aufgetragen wird.

**9.** Verwendung gemäß Anspruch 7, wobei die Zusammensetzung etwa 5 Minuten vor bis etwa 5 Minuten nach dem Kontakt mit dem HIV aufgetragen wird.

**10.** Verwendung gemäß Anspruch 1, wobei es sich bei dem HIV um bestimmte Stämme handelt.

**11.** Verwendung gemäß Anspruch 1, wobei es sich bei der Verbindung um Verbindung 96 handelt.

**12.** Verwendung gemäß Anspruch 1, wobei die Zusammensetzung mit einem Applikator aufgetragen wird.

**13.** Verwendung gemäß Anspruch 12, wobei es sich bei dem Applikator um ein Kondom handelt.

**14.** Verfahren zum Desinfizieren einer Flüssigkeit, die ein HIV enthält, umfassend das Zugeben einer Zusammensetzung, die eine HIV-prophylaktisch wirksame Menge einer Verbindung mit der Struktur

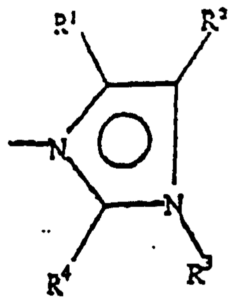

IIa

umfasst, zu der Flüssigkeit, wobei $R^1$, $R^2$, $R^3$ und $R^4$ gleich oder verschieden sein können und Wasserstoff oder Niederalkyl mit 1 bis 4 Kohlenstoffatomen sein können;
mit der Maßgabe, dass $R^1$, $R^2$, $R^3$ und $R^4$ ausreichend klein sind, so dass sie die Bindung des axialen Liganden an das Co-Atom nicht aufgrund von sterischer Hinderung verhindern.

**15.** Verfahren gemäß Anspruch 14, wobei die Verbindung in einer Menge von etwa 0,00005 bis etwa 5 Gew.-% der Flüssigkeit zugegeben wird.

**16.** Verfahren gemäß Anspruch 14, wobei die Verbindung in einer Menge von etwa 0,005 bis etwa 5 Gew.-% der Flüssigkeit zugegeben wird.

**17.** Verfahren gemäß Anspruch 14, wobei die Verbindung in einer Menge von etwa 0,005 bis etwa 2 Gew.-% der Flüssigkeit zugegeben wird.

**18.** Verfahren gemäß Anspruch 14, wobei die Verbindung in einer Menge von etwa 0,01 bis etwa 2 Gew.-% der Flüssigkeit zugegeben wird.

**19.** Verfahren gemäß Anspruch 14, wobei die Flüssigkeit ein Wachstumsmedium oder ein von Blut abgeleitetes Produkt ist.

**20.** Verwendung einer Verbindung der Formel II zur Herstellung einer Zusammensetzung zur Prävention einer Infektion mit HPV (humanem Papillomvirus):

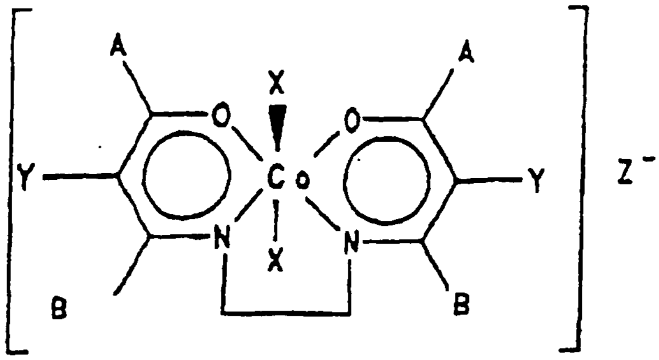

II

wobei jeweils

A gleich oder verschieden sein können und eine Alkylgruppe, eine Phenylgruppe oder ein substituiertes Derivat einer Phenylgruppe bedeuten;

Y gleich oder verschieden sein können und Wasserstoff, eine unverzweigte Alkylgruppe, ein Halogenid oder eine Gruppe mit der Struktur

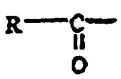

bedeuten, wobei R Wasserstoff, eine Alkoxidgruppe, eine Alkylgruppe oder OH ist;

B x gleich oder verschieden sein können und jeweils Wasserstoff oder eine Alkylgruppe sind;

$Z^-$ ein lösliches, pharmazeutisch annehmbares negatives Ion ist; und

X gleich oder verschieden sein können und einen axialen Liganden bedeuten, der aus der Gruppe ausgewählt ist, die aus Struktureinheiten mit der Formel

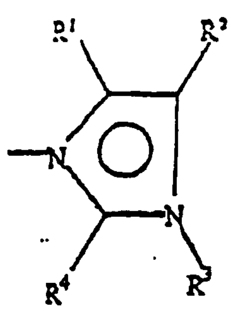

IIa

besteht, wobei $R^1$, $R^2$, $R^3$ und $R^4$ gleich oder verschieden sein können und Wasserstoff oder Niederalkyl mit 1 bis 4 Kohlenstoffatomen sein können;
mit der Maßgabe, dass $R^1$, $R^2$, $R^3$ und $R^4$ ausreichend klein sind, so dass sie die Bindung des axialen Liganden an das Co-Atom nicht aufgrund von sterischer Hinderung verhindern.

**21.** Verwendung gemäß Anspruch 20, wobei die Verbindung etwa 0,00005 bis etwa 5 Gew.-% der Zusammensetzung ausmacht.

**22.** Verwendung gemäß Anspruch 20, wobei die Verbindung etwa 0,005 bis etwa 5 Gew.-% der Zusammensetzung ausmacht.

**23.** Verwendung gemäß Anspruch 20, wobei die Verbindung etwa 0,005 bis etwa 2 Gew.-% der Zusammensetzung ausmacht.

**24.** Verwendung gemäß Anspruch 20, wobei die Verbindung etwa 0,01 bis etwa 2 Gew.-% der Zusammensetzung ausmacht.

**25.** Verwendung gemäß Anspruch 20, wobei die Zusammensetzung in Form einer pharmazeutisch annehmbaren Kochsalzlösung, einer Salbe, eines Balsams, einer Creme oder dergleichen vorliegt.

**26.** Verwendung gemäß Anspruch 20, wobei die Zusammensetzung auf diejenige Stelle aufgetragen wird, die dem HPV ausgesetzt ist.

**27.** Verwendung gemäß Anspruch 26, wobei die Zusammensetzung etwa 1 Stunde vor bis etwa 6 Stunden nach dem Kontakt mit dem HPV aufgetragen wird.

**28.** Verwendung gemäß Anspruch 26, wobei die Zusammensetzung etwa 5 Minuten vor bis etwa 5 Minuten nach dem Kontakt mit dem HPV aufgetragen wird.

**29.** Verwendung gemäß Anspruch 20, wobei das HPV aus der Gruppe ausgewählt ist, die aus HPV-1, HPV-2, HPV-3, HPV-4, HPV-6, HPV-7, HPV-10, HPV-11, HPV-16, HPV-18, HPV-31 oder HPV-45 besteht.

**30.** Verwendung gemäß Anspruch 20, wobei es sich bei der Verbindung um CTC 96 handelt.

**31.** Verwendung gemäß Anspruch 20, wobei die Zusammensetzung mit Hilfe eines mit der Zusammensetzung beschichteten Applikators aufgetragen wird.

**32.** Verwendung gemäß Anspruch 31, wobei es sich bei dem Applikator um ein Kondom handelt.

**33.** Verfahren zum Desinfizieren einer Flüssigkeit, die ein HPV enthält, umfassend das Zugeben einer Zusammensetzung, die eine HPV-prophylaktisch wirksame Menge einer Verbindung mit der Struktur

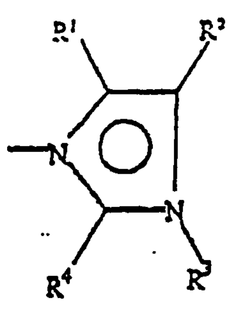

IIa

umfasst, zu der Flüssigkeit, wobei $R^1$, $R^2$, $R^3$ und $R^4$ gleich oder verschieden sein können und Wasserstoff oder Niederalkyl mit 1 bis 4 Kohlenstoffatomen sein können;
mit der Maßgabe, dass $R^1$, $R^2$, $R^3$ und $R^4$ ausreichend klein sind, so dass sie die Bindung des axialen Liganden an das Co-Atom nicht aufgrund von sterischer Hinderung verhindern.

**34.** Verfahren gemäß Anspruch 33, wobei die Verbindung in einer Menge von etwa 0,00005 bis etwa 5 Gew.-% der Flüssigkeit zugegeben wird.

**35.** Verfahren gemäß Anspruch 33, wobei die Verbindung in einer Menge von etwa 0,005 bis etwa 5 Gew.-% der Flüssigkeit zugegeben wird.

**36.** Verfahren gemäß Anspruch 33, wobei die Verbindung in einer Menge von etwa 0,005 bis etwa 2 Gew.-% der Flüssigkeit zugegeben wird.

**37.** Verfahren gemäß Anspruch 33, wobei die Verbindung in einer Menge von etwa 0,01 bis etwa 2 Gew.-% der Flüssigkeit zugegeben wird.

**38.** Verfahren gemäß Anspruch 33, wobei die Flüssigkeit ein Wachstumsmedium oder ein von Blut abgeleitetes Produkt ist.

**Revendications**

**1.** Utilisation d'un composé de formule II pour la fabrication d'une composition pour la prévention de l'infection par le virus de l'immunodéficience humaine :

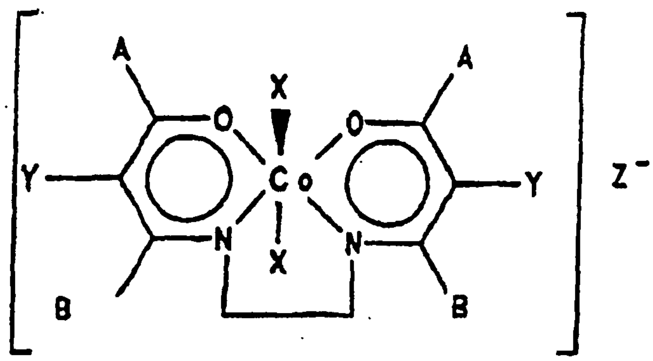

II

dans laquelle chaque

A peut être identique ou différent et est un groupe alkyle, un groupe phényle ou un dérivé substitué d'un groupe phényle ;

Y peut être identique ou différent et est hydrogène, un groupe alkyle non ramifié, un halogénure ou un groupe ayant la structure

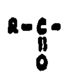

dans laquelle R est hydrogène, un groupe alcoxyde, un groupe alkyle, ou OH ;

B peut être identique ou différent et chacun est hydrogène ou un groupe alkyle ;

$Z^-$ est un ion négatif soluble pharmaceutiquement acceptable, et

X peut être identique ou différent et est un ligand axial choisi dans le groupe comprenant des groupements de formule :

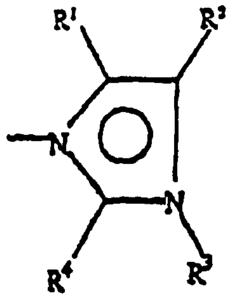

IIa

dans laquelle $R^1$, $R^2$, $R^3$ et $R^4$ peuvent être identiques ou différents et peuvent être hydrogène ou un alkyle inférieur ayant 1 à 4 atomes de carbone ;
à condition que $R^1$, $R^2$, $R^3$ et $R^4$ soient de taille suffisamment petite pour ne pas empêcher la fixation du ligand axial à l'atome Co par encombrement stérique.

**2.** Utilisation selon la revendication 1 dans laquelle le composé constitue environ 0,00005 à environ 5 % en poids de la composition.

**3.** Utilisation selon la revendication 1 dans laquelle le composé constitue environ 0,005 à environ 5 % en poids de la composition.

**4.** Utilisation selon la revendication 1 dans laquelle le composé constitue environ 0,005 à environ 2 % en poids de la composition.

**5.** Utilisation selon la revendication 1 dans laquelle le composé constitue environ 0,01 à environ 2 % en poids de la composition.

**6.** Utilisation selon la revendication 1 dans laquelle la composition est sous la forme d'une solution saline, d'un onguent, d'une pommade, d'une crème, ou autres similaires pharmaceutiquement acceptables.

**7.** Utilisation selon la revendication 1 dans laquelle la composition est appliquée sur le site qui est exposé au virus de l'immunodéficience humaine.

**8.** Utilisation selon la revendication 7 dans laquelle la composition est appliquée à partir d'environ une heure avant à environ six heures après l'exposition au virus de l'immunodéficience humaine.

**9.** Utilisation selon la revendication 7 dans laquelle la composition est appliquée à partir d'environ cinq minutes avant à environ cinq minutes après l'exposition au virus de l'immunodéficience humaine.

**10.** Utilisation selon la revendication 1 dans laquelle le virus de l'immunodéficience humaine est le virus des SOUCHES ???

**11.** Utilisation selon la revendication 1 dans laquelle le composé est le composé 96.

**12.** Utilisation selon la revendication 1 dans laquelle la composition est appliquée sur un applicateur.

**13.** Utilisation selon la revendication 12 dans laquelle l'applicateur est un préservatif.

**14.** Procédé pour désinfecter un liquide contenant un virus de l'immunodéficience humaine comprenant l'étape consistant à ajouter au liquide une composition comprenant une quantité efficace pour la prophylaxie du virus de l'immunodéficience humaine d'un composé ayant la structure

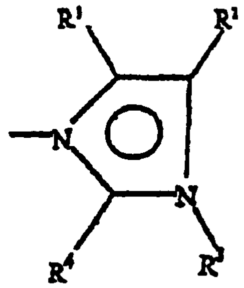

IIa

dans laquelle $R^1$, $R^2$, $R^3$ et $R^4$ peuvent être identiques ou différents et peuvent être hydrogène ou un alkyle inférieur ayant 1 à 4 atomes de carbone ;
à condition que $R^1$, $R^2$, $R^3$ et $R^4$ soient de taille suffisamment petite pour ne pas empêcher la fixation du ligand axial à l'atome Co par encombrement stérique.

**15.** Procédé selon la revendication 14 dans lequel le composé est ajouté en une quantité d'environ 0,00005 à environ 5 % en poids du liquide.

**16.** Procédé selon la revendication 14 dans lequel le composé est ajouté en une quantité d'environ 0,005 à environ 5 % en poids du liquide.

**17.** Procédé selon la revendication 14 dans lequel le composé est ajouté en une quantité d'environ 0,005 à environ 2 % en poids du liquide.

**18.** Procédé selon la revendication 14 dans lequel le composé est ajouté en une quantité d'environ 0,01 à environ 2 % en poids du liquide.

**19.** Procédé selon la revendication 14 dans lequel le liquide est un milieu de croissance ou un produit dérivé du sang.

**20.** Utilisation d'un composé de formule II pour la fabrication d'une composition pour la prévention de l'infection par le papillomavirus humain

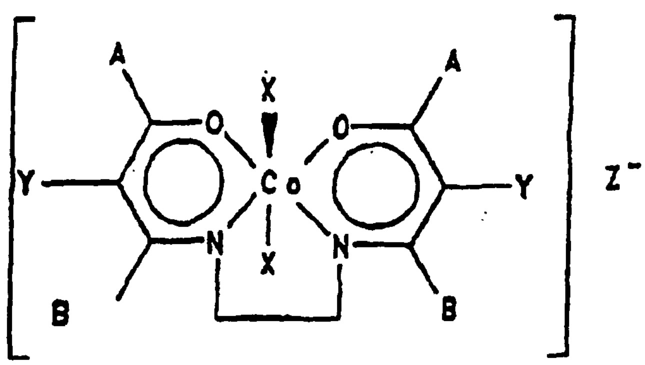

II

dans laquelle chaque

A peut être identique ou différent et est un groupe alkyle, un groupe phényle ou un dérivé substitué d'un groupe phényle ;
Y peut être identique ou différent et est hydrogène, un groupe alkyle non ramifié, un halogénure ou un groupe

ayant la structure

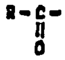

dans laquelle R est hydrogène, un groupe alcoxyde, un groupe alkyle, ou OH ;

B peut être identique ou différent et chacun est hydrogène ou un groupe alkyle ;

$Z^-$ est un ion négatif soluble pharmaceutiquement acceptable, et

X peut être identique ou différent et est un ligand axial choisi dans le groupe comprenant des groupements de formule :

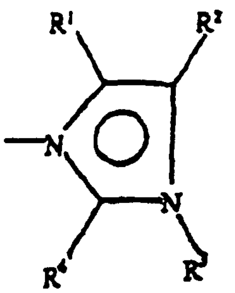

IIa

dans laquelle $R^1$, $R^2$, $R^3$ et $R^4$ peuvent être identiques ou différents et peuvent être hydrogène ou un alkyle inférieur ayant 1 à 4 atomes de carbone ;

à condition que $R^1$, $R^2$, $R^3$ et $R^4$ soient de taille suffisamment petite pour ne pas empêcher la fixation du ligand axial à l'atome Co par encombrement stérique.

**21.** Utilisation selon la revendication 20 dans laquelle le composé constitue environ 0,00005 à environ 5 % en poids de la composition.

**22.** Utilisation selon la revendication 20 dans laquelle le composé constitue environ 0,005 à environ 5 % en poids de la composition.

**23.** Utilisation selon la revendication 20 dans laquelle le composé constitue environ 0,005 à environ 2 % en poids de la composition.

**24.** Utilisation selon la revendication 20 dans laquelle le composé constitue environ 0,01 à environ 2 % en poids de la composition.

**25.** Utilisation selon la revendication 20 dans laquelle la composition est sous la forme d'une solution saline, d'un onguent, d'une pommade, d'une crème ou autres similaires pharmaceutiquement acceptables.

**26.** Utilisation selon la revendication 20 dans laquelle la composition est appliquée au site qui est exposé au papillomavirus humain.

**27.** Utilisation selon la revendication 26 dans laquelle la composition est appliquée à partir d'environ 1 heure avant à environ 6 heures après exposition au papillomavirus humain.

**28.** Utilisation selon la revendication 26 dans laquelle la composition est appliquée à partir d'environ 5 minutes avant à environ 5 minutes après exposition au papillomavirus humain.

**29.** Utilisation selon la revendication 20 dans laquelle le papillomavirus humain est choisi dans le groupe comprenant HPV-1, HPV-2, HPV-3, HPV-4, HPV-6, HPV-7, HPV-10, HPV-11, HPV-16, HPV-18, HPV-31 ou HPV-45.

**30.** Utilisation selon la revendication 20 dans laquelle le composé est CTC96.

**31.** Utilisation selon la revendication 20 dans laquelle la composition est appliquée sur un applicateur enrobé de la composition.

**32.** Utilisation selon la revendication 31 dans laquelle l'applicateur est un préservatif.

**33.** Procédé pour désinfecter un liquide contenant un papillomavirus humain comprenant l'étape consistant à ajouter au liquide une composition comprenant une quantité efficace pour la prophylaxie du papillomavirus humain d'un composé de formule :

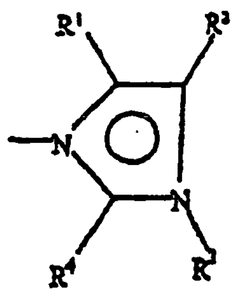

IIa

dans laquelle $R^1$, $R^2$, $R^3$ et $R^4$ peuvent être identiques ou différents et peuvent être hydrogène ou un alkyle inférieur ayant 1 à 4 atomes de carbone ;
à condition que $R^1$, $R^2$, $R^3$ et $R^4$ soient de taille suffisamment petite pour ne pas empêcher la fixation du ligand axial à l'atome Co par encombrement stérique.

**34.** Procédé selon la revendication 33 dans lequel le composé est ajouté en une quantité d'environ 0,00005 à environ 5 % en poids du liquide.

**35.** Procédé selon la revendication 33 dans lequel le composé est ajouté en une quantité d'environ 0,005 à environ 5 % en poids du liquide.

**36.** Procédé selon la revendication 33 dans lequel le composé est ajouté en une quantité d'environ 0,005 à environ 2 % en poids du liquide.

**37.** Procédé selon la revendication 33 dans lequel le composé est ajouté en une quantité d'environ 0,01 à environ 2 % en poids du liquide.

**38.** Procédé selon la revendication 33 dans lequel le liquide est un milieu de croissance ou un produit dérivé du sang.

Fig. 1

Scatterplot of the Graft Composite Geometric Mean Diameter